(19) Europäisches Patentamt
European Patent Office
Office européen des brevets

(11) Veröffentlichungsnummer: **0 036 521**
**B1**

(12) **EUROPÄISCHE PATENTSCHRIFT**

(45) Veröffentlichungstag der Patentschrift: **19.10.83**

(51) Int. Cl.³: **C 07 D 233/06**, C 07 D 233/08, C 07 D 233/10

(21) Anmeldenummer: **81101536.1**

(22) Anmeldetag: **04.03.81**

(54) **Verfahren zur Herstellung von 2-Imidazolinen.**

(30) Priorität: **13.03.80 DE 3009633**

(43) Veröffentlichungstag der Anmeldung:
**30.09.81 Patentblatt 81/39**

(45) Bekanntmachung des Hinweises auf die Patenterteilung:
**19.10.83 Patentblatt 83/42**

(84) Benannte Vertragsstaaten:
**BE CH DE FR GB LI**

(56) Entgegenhaltungen:
**EP-A-0 000 208**
**EP-A-0 012 371**
**DE-A-1 670 400**
**CHEMICAL ABSTRACTS, Band 86, Nr. 19, 9. Mai 1977, Seite 566, Nr. 140048x, Columbus, Ohio, USA, S. A. ZELENAYA et al.: »Imidazolines«**

(73) Patentinhaber: **BASF Aktiengesellschaft, Carl-Bosch-Strasse 38, D-6700 Ludwigshafen (DE)**

(72) Erfinder: **Dockner, Toni, Dr., Grossgasse 6, D-6701 Meckenheim (DE)**
Erfinder: **Kempe, Uwe, Dr., Carl-Bosch-Strasse 44, D-6703 Limburgerhof (DE)**
Erfinder: **Krug, Herbert, Mussbacher Strasse 49, D-6700 Ludwigshafen (DE)**
Erfinder: **Magnussen, Peter, Dr., Professor-Dillinger-Weg 25, D-6702 Bad Duerkheim 1 (DE)**
Erfinder: **Praetorius, Werner, Dr., Dirmsteiner Weg 47, D-6700 Ludwigshafen (DE)**

**EP 0 036 521 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von 2-Imidazolinen

Die Erfindung betrifft ein neues Verfahren zur Herstellung von 2-Imidazolinen durch Umsetzung von N,N'-Diformyl-1,2-diaminen bei 200 bis 350°C in Gegenwart bestimmter Mengen an Inertgas und von Zinkoxid bestimmter Struktur oder einem Gemisch von diesem Zinkoxid und Aluminiumoxid als Katalysatoren in der Gasphase.

Die Umsetzung von Formaldehyd mit Diaminoalkanen in Gegenwart von Schwefel führt zu 2-Imidazolinen, wobei allerdings Schwefelwasserstoff in stöchiometrischen Mengen gebildet wird (DE-A-2 132 079). In der DE-C-1 189 998 wird die Synthese von 2-Imidazolinen durch Umsetzung von Diaminoalkanen mit Blausäure beschrieben. Neben zum Teil unbefriedigenden Ausbeuten hat dieses Verfahren den Nachteil, daß bei großtechnischen Prozessen mit Blausäure gearbeitet werden muß.

Die Reaktion von Diaminoalkanen mit tert.-Butylisonitril zu 4,5-Dihydroimidazol in Gegenwart von Silbercyanid ist ebenfalls bekannt [J. Amer. Chem. Soc., B. 95, S. 4447, 4448 (1973)]. Die Ausbeuten sind befriedigend, jedoch ist tert.-Butylisonitril geruchsbelästigend und für eine Anwendung dieses Verfahrens in größerem Maßstab schon aus Gründen der Betriebssicherheit und Umweltschutz ungeeignet.

Es ist aus der DE-A-1 922 802 bekannt, daß man N,N'-Diformyl-1,2-diaminoalkane bei 400 bis 600°C an aktivierter Kieselsäure oder Silikaten im Festbettreaktor bei Drücken von zweckmäßig 1 bis 100 mm Hg zu 2-Imidazolinen umsetzen kann. Nachteilig an diesem Verfahren sind jedoch die niedrigen Drucke und die hohe Zersetzlichkeit von 2-Imidazolinen, die in der US-A-3 629 278 beschrieben wird. Ein wesentliches Merkmal des Verfahrens ist ein Reaktionsdruck von weniger als 200 mm Hg; beispielhaft arbeitet man bei einem Druck von 0,25 bis 100 mm Hg. Nachteilig ist jedoch wiederum der niedrige Druck, der eine kontinuierliche Herstellung von 2-Imidazolinen im großtechnischen Maßstab erschwert. Es wird angegeben, daß nach anderen Verfahren 2-Imidazoline nicht aus N,N'-Diformylalkylendiaminen hergestellt werden können.

Die EP-A-0 000 208 beschreibt ein Verfahren zur Synthese von Imidazolen durch Umsetzung von Carbonsäuren mit 1.2-Diaminen oder durch Umsetzung von 2-Imidazolinen bei Temperaturen von 300–600°C in Gegenwart von Zinkoxid oder Gemischen von Zinkoxid und Aluminiumoxid als Katalysatoren. Die Lebensdauer der Katalysatoren beträgt mehr als 300 bzw. 72 Stunden (Beispiele). Eine Rückführung der Abgase (Kreisgas) in die Reaktion wird nicht beschrieben.

Die EP-A-0 012 371 beschreibt ein Verfahren zur Herstellung von in 2-Stellung substituierten 2-Imidazolinen durch Umsetzung von 1.2-Diaminen mit Nitrilen oder Carbonylverbindungen bei Temperaturen von 200–450°C in Gegenwart von Oxiden oder Phosphaten der Metalle der 3. und 4. Gruppe des Periodischen Systems und/oder Siliciumdioxid in der Gasphase.

Die DE-A-1 670 400 beschreibt ein Verfahren zur Herstellung von 1-Aryl-2-alkyl-Δ2-Imidazolinen durch Umsetzung von N-Aryl-N'-acylethylendiaminen in der Gasphase unter vermindertem Druck bei Temperaturen von 100–400°C. Als Katalysator wird in den Beispielen bei dieser Reaktion basisches gekörntes Aluminiumoxid eingesetzt.

Chem. Abstr., Bd. 86, Nr. 19, S. 566, Nr. 140048, beschreibt die Synthese von Imidazolinen aus Ethylendiamin und Fettsäuren in Gegenwart von Aluminiumoxid bei Temperaturen von 260–280°C.

Es wurde nun gefunden, daß man 2-Imidazoline der allgemeinen Formel I

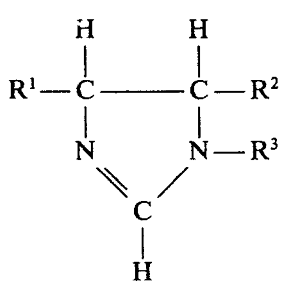

(I)

worin $R^1$, $R^2$ und $R^3$ gleich oder verschieden sein können und jeweils einen aliphatischen, araliphatischen oder aromatischen Rest oder ein Wasserstoffatom bedeuten, durch Umsetzung von N,N'-Diformylalkylendiaminen in Gegenwart von Metalloxidkatalysatoren vorteilhaft erhält, wenn man N,N'-Diformyl-1,2-diamine der allgemeinen Formel II

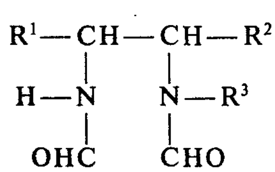

(II)

worin $R^1$, $R^2$ und $R^3$ die vorgenannte Bedeutung besitzen, bei einer Temperatur von 200 bis 350°C in Gegenwart von Inertgasen in einem Verhältnis von 5 bis 40 Mol Inertgas je Mol Ausgangsstoff II und von Zinkoxid mit einem Porenvolumen von 0,05 bis 1 Milliliter je Gramm und einer spezifischen Oberfläche von 1 bis 500 Quadratmeter je Gramm oder einem Gemisch von diesem Zinkoxid des vorgenannten Porenvolumens und der vorgenannten spezifischen Oberfläche und Aluminiumoxid oder einem Gemisch von diesem Zinkoxid des vorgenannten Porenvolumens und der vorgenannten spezifischen Oberfläche und Eisen(III)-oxid als Katalysatoren in der Gasphase umsetzt.

Die Umsetzung kann für den Fall der Verwendung von N,N'-Diformyl-1,2-diaminopropan durch die folgenden Formeln wiedergegeben werden:

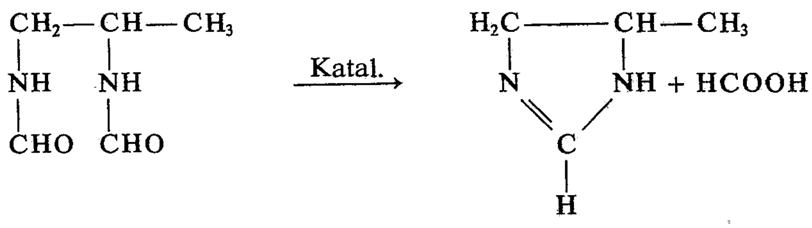


Im Vergleich zu den bekannten Verfahren liefert das Verfahren nach der Erfindung auf einfacherem und wirtschaftlicherem Wege 2-Imidazoline in teilweise besserer Ausbeute und Reinheit. Schwefel- und Blausäureverbindungen als Reaktionskomponenten werden vermieden, jedoch überraschend hohe Ausbeuten erzielt. Bei Unterdruck muß nicht umgesetzt werden. Vorteilhaft ist die Arbeitsweise in der Gasphase; so können 2-Imidazoline, die in flüssiger Phase unbeständig sind, auch ohne Isolierung weiter umgesetzt werden. Die Dehydrierung des wenig stabilen 2-Imidazolins zum stabilen Imidazol kann beispielsweise leicht in der Gasphase in einer zweiten Reaktionsstufe erfolgen. Alle diese vorteilhaften Ergebnisse erlauben gerade auch einen großtechnischen, kontinuierlichen Betrieb bei langer Lebensdauer des Katalysators und sind im Hinblick auf den Stand der Technik überraschend. Man hätte im Hinblick auf die US-A-3 629 278 und auf die DE-A-1 922 802 angesichts der andersartigen Katalysatoren keine Reaktion oder zumindest hohe Ausbeuteverluste erwarten müssen. Insbesondere mußte im Hinblick auf die DE-C-1 231 249, die EP-A-0 000 208 und die DE-A-2 729 017 angenommen werden, daß im wesentlichen Imidazole als Endstoffe erhalten werden. Überraschend war es auch im Vergleich mit dem in der DE-C-1 231 249 beschriebenen Verfahren (Beispiel 4), daß an Zinkoxid der erfindungsgemäßen Struktur bei Temperaturen über 250°C anstelle von Imidazol 2-Imidazolin in hoher Ausbeute entsteht. Im Hinblick auf DE-A-2 729 017 und EP-A-0 000 208, Beispiel 2, andererseits war ebenfalls nicht zu vermuten, daß die Verwendung der erfindungsgemäßen Ausgangsstoffe II anstelle von Äthylendiamin und Ameisensäure auch bei Temperaturen über 300°C anstelle von Imidazol Imidazolin in hoher Ausbeute liefern würde. Im Hinblick auf EP-A-0 000 208 haben die erfindungsgemäßen Katalysatoren überraschend eine höhere Lebensdauer (Standzeiten in Beispielen: mehr als 1100 bzw. 1500 Stunden) und erlauben einen Betrieb mit Kreisgas. Auch aus EP-A-0 012 371, DE-A-1 670 400 und Chem. Abstr. (loc. cit.) konnte nicht abgeleitet werden, daß gerade die erfindungsgemäßen Ausgangsstoffe mit Zinkoxid spezieller Struktur hohe Ausbeuten an in 2-Stellung unsubstituierten Imidazolinen liefern.

Bevorzugte Ausgangsstoffe II und dementsprechend bevorzugte Endstoffe I sind solche, in deren Formeln $R^1$, $R^2$ und $R^3$ gleich oder verschieden sein können und jeweils einen Alkylrest mit 1 bis 18 Kohlenstoffatomen, insbesondere mit 1 bis 8 Kohlenstoffatomen, einen Alkenylrest mit mehreren oder insbesondere einer Doppelbindung und mit 2 bis 18, vorzugsweise 3 bis 18, insbesondere 4 bis 8 Kohlenstoffatomen, einen Aralkylrest oder Alkylarylrest mit 7 bis 12 Kohlenstoffatomen oder einen Phenylrest oder ein Wasserstoffatom bedeuten. Die vorgenannten Reste können noch durch unter den Reaktionsbedingungen inerte Gruppen, z. B. Alkylgruppen oder Alkoxygruppen mit jeweils 1 bis 4 Kohlenstoffatomen, substituiert sein.

Als Ausgangsstoffe II kommen z. B. in Frage: die N,N'-Diformylverbindungen von Äthylendiamin, 1,2-Propylendiamin, 1,2-Butylendiamin, 1,2-Pentylendiamin, 1,2-n-Hexylendiamin, 1,2-n-Heptylendiamin, 1,2-n-Octylendiamin, 1,2-n-Nonylendiamin, 1,2-n-Decylendiamin, 1,2-n-Octadecylendiamin; 2,3-Butylendiamin, 2,3-Pentylendiamin, 2,3-Hexylendiamin, 2,3-Heptylendiamin, 2,3-Octylendiamin, 2,3-Nonylendiamin, 2,3-Decylendiamin, 3,4-Hexylendiamin, 3,4-Heptylendiamin, 3,4-Octylendiamin, 3,4-Nonylendiamin, 3,4-Decylendiamin, 4,5-Octylendiamin, 4,5-Nonylendiamin, 4,5-Decylendiamin und 5,6-Decylendiamin; durch die Benzyl- und/oder Phenylgruppe in 1-Stellung einfach oder in 1- und 2-Stellung gleichzeitig substituierte Äthylendiamine; durch vorgenannte Alkylgruppen in 1-Stellung und durch die Benzyl- oder Phenylgruppe in 2-Stellung substituierte Äthylendiamine; die N-Methyl-, N-Äthyl-, N-Propyl-, N-Isopropyl-, N-Butyl-, N-Isobutyl-, N-sek.-Butyl-, N-tert.-Butyl-, N-Benzyl-, N-Phenyl-Verbindungen vorgenannter N,N'-Diformyl-1,2-diamine.

Die Umsetzung wird bei einer Temperatur von 200 bis 350°C, zweckmäßig von 250 bis 350°C, vorzugsweise von 260 bis 340°C, insbesondere von 270 bis 330°C, drucklos oder unter Druck, in der Regel bei mindestens 1 bar, zweckmäßig bei 1 bis 10, vorzugsweise 1 bis 3 bar, diskontinuierlich oder

zweckmäßig kontinuierlich durchgeführt. In der Regel dient das Reaktionsgemisch auch als Lösungsmedium, gegebenenfalls können auch unter den Reaktionsbedingungen inerte, organische, zweckmäßig mit Wasser kein Azeotrop bildende Lösungsmittel, z. B. aliphatische Kohlenwasserstoffe wie Petroläther oder Ligroin, verwendet werden. Unter den organischen Lösungsmitteln sind solche mit einem Siedepunkt über 120° C, zweckmäßig über 140° C, z. B. entsprechende Benzinfraktionen von 120 bis 160° C, bevorzugt.

Als Katalysator wird Zinkoxid mit einem Porenvolumen von 0,05 bis 1 Milliliter je Gramm und einer spezifischen Oberfläche von 1 bis 500 Quadratmeter je Gramm allein oder ein Gemisch von diesem Zinkoxid und Aluminiumoxid, zweckmäßig in einem Verhältnis von Zink zu Aluminium wie 1 bis 50, vorzugsweise 8 bis 10 Grammatom Zink je Grammatom Aluminium, und von 0,1 bis 1, vorzugsweise von 0,2 bis 0,4 Grammatom Zink je Mol Ausgangsstoff II verwendet. Als Aluminiumoxid kommen z. B. $\alpha$- und $\gamma$-Aluminiumoxid in Frage. Anstelle von Aluminiumoxid kommen auch dieses Oxid enthaltende Stoffe oder Stoffgemische in Betracht, z. B. Aluminiumsilikat, Magnesiumaluminiumsilikathydrat, Dimagnesiumaluminiumsilikathydrat, Natriumaluminiumsilikat, Calciumaluminiumsilikat, Fullererde, Tone, Bleicherden wie Bentonit, Bauxit, Bimsstein, Andalusit, Kaolin, Allophane, Zeolithe, Mullit, Korund, $\gamma$-Tonerde, Hydrargillit und Böhmit.

Der Katalysator kann trägerfrei sein oder auch auf einem Träger, vorteilhaft in einer Menge von 1 bis 18 Gewichtsprozent Katalysator, bezogen auf den Träger, aufgebracht sein. Ebenfalls können vorgenannte Aluminiumverbindungen gleichzeitig in Gestalt des darin enthaltenen $Al_2O_3$ als Katalysatorkomponente und für das Zinkoxid als Träger dienen. Als Träger kommen zweckmäßig in Frage Kieselsäureverbindungen wie Silikate, z. B. Montmorillonit, Floridaerde, Quarz, Asbest; gefällte Kieselsäure, Kieselgel, Kieselgur; Titandioxid, Zirkondioxid, Zinndioxid, Aktivkohle; Erdalkalisulfate oder Erdalkaliphosphate, z. B. die Calcium- oder Bariumsalze; oder entsprechende Gemische vorgenannter Trägermaterialien. Die Herstellung der Trägerkatalysatoren wird nach den üblichen Verfahren, z. B. durch Auftragen der Zinkverbindung und gegebenenfalls der Aluminiumverbindung auf den Träger, Trocknen und Calcinieren, beispielsweise zwischen 400 und 1200° C in reduzierender, oxidierender oder inerter Atmosphäre, durchgeführt. Der Träger kann auch in seiner gewünschten geometrischen Form mit einer Lösung der Zinkverbindung allein bzw. der Zink- und Aluminiumverbindung getränkt und getrocknet werden. Ebenfalls kann man das Trägermaterial mit der Zinkverbindung und gegebenenfalls der Aluminiumverbindung und Wasser verkneten, in die gewünschte Form bringen, trocknen und bei einer Temperatur von 400 bis 1200° C calcinieren.

Die Teilchengröße der Katalysatoren beträgt vorzugsweise von 0,05 bis 7, insbesondere 2 bis 4 Millimeter. Die Form kann beliebig, z. B. in Pillen-, Zylinder- oder Strangform, kugelförmig oder körnig, gewählt werden. Der Katalysator hat Porenvolumina von 0,05 bis 1 Milliliter je Gramm, bevorzugt 0,1 bis 0,8 Milliliter je Gramm, spezifische Oberflächen von 1 bis 500 Quadratmeter je Gramm, bevorzugt 30 bis 150 Quadratmeter je Gramm. Der Katalysator hat bevorzugt Schüttgewichte von 0,4 bis 2,1 Gramm je Milliliter. Als spezifische Gesamtoberfläche wird die gesamte innere und äußere Oberfläche des Katalysators, bezogen auf 1 Gramm Katalysator, verstanden. Zur Bestimmung der spezifischen Gesamtoberfläche können die üblichen Methoden zur Bestimmung der Gesamtoberfläche von Katalysatoren, z. B. die BET-Methode (Ullmanns Encyklopädie der technischen Chemie, Band 9, Seite 266), herangezogen werden. Im allgemeinen kommt ein Porenradius des Katalysators von 1,5 bis 10, vorteilhaft von 3 bis 8 Nanometern, in Betracht. Man kann auch mit Katalysator überzogene Rohre oder netzartige Träger verwenden.

Vorzugsweise werden die Katalysatoren in Splitt- oder Kugelform in der Wirbelschicht eingesetzt, wobei zweckmäßig Katalysatorteilchen mit Korngrößen von 0,005 bis 3 mm, insbesondere von 0,1 bis 1 mm, bevorzugt 0,2 bis 0,4 mm, verwendet werden. Die Schichthöhe des Katalysatorbettes im Wirbelzustand beträgt vorteilhaft 30 bis 3000 Milliliter oder wird zweckmäßig so gewählt, daß sich Verweilzeiten der Ausgangsstoffe II in der Katalysatorschicht von 0,01 bis 20, vorzugsweise von 1 bis 10 Sekunden ergeben. Bezüglich der Herstellung der Katalysatoren wird auf Houben—Weyl, Methoden der Organischen Chemie, Band 4/2, Seiten 142 ff., und Ullmanns Encyklopädie der technischen Chemie, Band 9, Seiten 271 ff., verwiesen.

Gegebenenfalls können neben dem Zinkoxid als Hilfskatalysatoren noch andere Oxide Verwendung finden, z. B. Eisen(III)-oxid; zweckmäßig sind Mengen von 0,2 bis 2, insbesondere 0,8 bis 1,2 Mol Fe(III)-oxid je Mol Zinkoxid. Vorteilhaft ist eine Kombination von Zinkoxid und Eisen(III)-oxid auf Siliciumdioxidträger, z. B. Kieselsäure oder Kieselgel, zweckmäßig in einem Verhältnis von 12 bis 20 Gewichtsprozent $Fe_2O_3$, bezogen auf $SiO_2$, und 150 bis 250 Gewichtsprozent $Fe_2O_3$, bezogen auf ZnO.

Als unter den Reaktionsbedingungen inerte Gase (Inertgas) werden zweckmäßig Edelgase wie Xenon, Argon, Neon, Helium; Alkane wie Methan, Äthan, Propan, 2,2-Dimethylpropan, Butan, Pentan, Isobutan; bevorzugt Stickstoff, Kohlenmonoxid und/oder Kohlendioxid; und entsprechende Gemische; verwendet. Vorteilhaft kann man auch das Abgas der erfindungsgemäßen Umsetzung zurückführen und dieses Kreisgas als Inertgas verwenden. Kreisgase enthalten zweckmäßig 0 bis 80 Gewichtsprozent Stickstoff, 5 bis 20 Gewichtsprozent $CO_2$, 5 bis 90 Gewichtsprozent Kohlenmonoxid, 0,1 bis 3 Gewichtsprozent Wasserstoff, 2,5 bis 10 Gewichtsprozent Wasserdampf, 0,01 bis 2 Gewichtsprozent aus dem Ausgangsstoff II entstandenes Alkan, 0,01 bis 2 Gewichtsprozent aus dem Ausgangsstoff II entstandenes Alken, 0,2 bis 2,5 Gewichtsprozent Ammoniak. Es kommen

vorzugsweise von 7 bis 38, insbesondere von 9 bis 36 Mol Inertgas je Mol Ausgangsstoff II in Betracht. Unter Mol Inertgas wird im Falle von Gasgemischen das durchschnittliche Mol des Gemischs verstanden.

Die Reaktion kann wie folgt durchgeführt werden: Der dampfförmige Ausgangsstoff II, im Gemisch mit Inertgas, wird bei der Reaktionstemperatur über den Katalysator bzw. Katalysator auf dem Träger in einem Röhren- oder Wirbelschichtreaktor geleitet. Eine Verweilzeit von 1 bis 40, insbesondere 1 bis 20 Sekunden im Reaktionsraum, ist vorteilhaft. Aus dem den Reaktor verlassenden Reaktionsgemisch wird der Endstoff in üblicher Weise, z. B. durch fraktionierte Destillation, isoliert. Man kann aber auch dem Reaktionsgemisch Proben entnehmen, durch analytische, z. B. gaschromatographische Bestimmung des Verhältnisses von Endstoff I und Ausgangsstoff II im Reaktionsgemisch den Umsatz feststellen und das Reaktionsgemisch ohne Abtrennung des Endstoffs direkt weiterverarbeiten, z. B. zu den entsprechenden Imidazolen.

In einer bevorzugten Ausführungsform des Verfahrens werden die Ausgangsstoffe in einer Wirbelschicht bei der Reaktionstemperatur umgesetzt. Der Katalysator bzw. Katalysator auf Träger kann zweckmäßig durch Inertgas oder dem Gemisch von Ausgangsstoff II und Inertgas als Wirbelschichtgas bei erhöhtem Druck in einer Wirbelschicht gehalten werden. Der Ausgangsstoff kann auch in einem beheizten Vorratsgefäß flüssig gehalten und in einen Verdampfer, der dem Wirbelschichtreaktor vorgeschaltet ist, dosiert werden. Gleichzeitig leitet man vorteilhaft einen Inertgasstrom, zweckmäßig von 5000 bis 50 000 Volumenteilen Inertgas je Stunde, durch den Verdampfer. Die verdampften Ausgangsstoffe werden zusammen mit dem Inertgasstrom durch das Katalysatorbett geleitet. Man kann das Verfahren nach der Erfindung in einem einfachen oder unterteilten, offenen oder geschlossenen Wirbelschichtsystem mit und ohne Fließstaubzirkulation durchführen. Bezüglich Reaktoren, Durchführung, Verfahrensvarianten und Reaktionsbedingungen des Wirbelschichtverfahrens wird auf Ullmanns Encyklopädie der technischen Chemie, Bd. 1, S. 916 ff., verwiesen. Die Aufarbeitung des Reaktionsgemisches erfolgt in vorgenannter Weise.

Die nach dem Verfahren der Erfindung herstellbaren 2-Imidazoline I sind wertvolle Ausgangsstoffe für die Herstellung von Farbstoffen, Pflanzenschutzmitteln und Pharmazeutika. 2-Imidazoline I werden als Katalysatoren für Polymerisationsreaktionen und Aldolkondensationen eingesetzt. Durch Dehydrierung an Aluminium-/Zinkoxidkatalysatoren liefern sie die entsprechenden Imidazole. Bezüglich der Verwendung wird auf die vorgenannten Veröffentlichungen und Ullmanns Encyklopädie der technischen Chemie, Bd. 13, S. 331 und 338, verwiesen.

Die in den folgenden Beispielen aufgeführten Teile bedeuten Gewichtsteile. Die Gewichtsteile verhalten sich zu den Volumenteilen wie Kilogramm zu Liter.

## Beispiel 1

56 Teile N,N'-Diformyl-1,2-diaminoäthan werden pro Stunde aus einem Vorratsgefäß in einen auf 300°C erhitzten horizontalen Quarzverdampfer dosiert und der Dampf zusammen mit 400 Volumenteilen pro Stunde Stickstoff durch den auf 300°C erhitzten Wirbelreaktor geleitet. Der Wirbelreaktor ist ein vertikal auf dem Verdampfer sitzendes, elektrisch beheiztes Quarzrohr, das nach unten mit einer eingeschmolzenen Quarzfritte abgeschlossen ist. Das Quarzrohr ist mit 220 Teilen eines Katalysators (Schüttgewicht 1,3 Gramm je Milliliter) aus 10 Gewichtsprozent ZnO und 90 Gewichtsprozent $\gamma$-Aluminiumoxid (Porenvolumen 0,921 Milliliter je Gramm; spezifische Oberfläche 83,3 Quadratmeter je Gramm; Porenradius 8 Nanometer) zur Hälfte gefüllt. Die Verweilzeit in der Katalysatorzone im Wirbelzustand beträgt 1,0 Sekunden. Die Höhe der Katalysatorzone beträgt im Wirbelzustand 150 mm. Die den Reaktor verlassenden Dämpfe werden kondensiert und fraktioniert destilliert. Man erhält stündlich 31,4 Teile (92,9% der Theorie) 2-Imidazolin vom Kp. 105°C (32 mbar). Der Umsatz ist praktisch quantitativ. Die Ausbeute bleibt auch noch nach 1500 Stunden Betrieb konstant.

## Beispiel 2

Man verfährt wie bei Beispiel 1, jedoch werden 220 Teile Zinkoxid (Struktur analog Beispiel 1) eingesetzt. Man erhält stündlich 27 Teile (79,8% der Theorie) 2-Imidazolin vom Kp. 105°C (32 mbar). Der Umsatz ist praktisch quantitativ. Die Ausbeute bleibt auch noch nach 1500 Stunden Betrieb konstant.

## Beispiel 3

Man verfährt wie bei Beispiel 1, jedoch werden 220 Teile Katalysator (Schüttgewicht 0,65 Gramm je Milliliter) Zinkoxid (Struktur analog Beispiel 1)-Aluminiumoxid (Gewichtsverhältnis 1 : 4) eingesetzt. Man erhält stündlich 29,8 Teile (88,1% der Theorie) 2-Imidazolin vom Kp. 105°C (32 mbar). Der Umsatz

ist praktisch quantitativ. Die Ausbeute bleibt auch noch nach 1500 Stunden Betrieb konstant.

Beispiel 4

Man verfährt wie bei Beispiel 1, jedoch werden 220 Teile Katalysator (Schüttgewicht 0,9 Gramm je Milliliter) Zinkoxid (Struktur analog Beispiel 1)-Aluminiumoxid (Gewichtsverhältnis 1 : 1) eingesetzt. Man erhält stündlich 29,4 Teile (87% der Theorie) 2-Imidazolin vom Kp. 105° C (32 mbar). Der Umsatz ist praktisch quantitativ. Die Ausbeute bleibt auch noch nach 1500 Stunden Betrieb konstant.

Beispiel 5

Man verfährt wie bei Beispiel 1, jedoch werden 220 Teile Katalysator (Schüttgewicht 0,8 Gramm je Milliliter) (6,6 Gewichtsprozent ZnO und 13 Gewichtsprozent $Fe_2O_3$ auf Siliciumdioxid) (Porenvolumen 0,11 Milliliter je Gramm, spezifische Oberfläche 45 Quadratmeter je Gramm; Porenradius 7,4 Nanometer) eingesetzt. Stündlich werden 49,9 Teile N,N'-Diformyldiaminoäthan in die Katalysatorzone eingebracht. Man erhält stündlich 27,5 Teile (91,3% der Theorie) 2-Imidazolin vom Kp. 105° C (32 mbar). Der Umsatz ist praktisch quantitativ. Die Ausbeute bleibt auch noch nach 1100 Stunden Betrieb konstant.

Beispiel 6

Man verfährt wie bei Beispiel 1, jedoch werden 220 Teile Katalysator (Schüttgewicht 0,73 Gramm je Milliliter) Zinkoxid (Struktur analog Beispiel 1)-Aluminiumoxid (Gewichtsverhältnis 3 : 7) eingesetzt. Stündlich werden 70 Teile N,N'-Diformyl-1,2-diaminopropan bei 290° C in die Katalysatorzone eingebracht. Man erhält stündlich 40,3 Teile (89% der Theorie) 4(5)-Methyl-2-imidazolin vom Kp. 94° C (20 mbar). Der Umsatz ist praktisch quantitativ. Die Ausbeute bleibt auch noch nach 1500 Stunden Betrieb konstant.

Beispiel 7

Man verfährt wie bei Beispiel 1, jedoch werden 220 Teile Katalysator (Schüttgewicht 1,3 Gramm je Milliliter) Zinkoxid (Struktur analog Beispiel 1)-Aluminiumoxid (Gewichtsverhältnis 9 : 1) eingesetzt. Bei 320° C werden stündlich 70 Teile N,N'-Diformyl-1,2-diaminopropan umgesetzt. Man erhält stündlich 42 Teile (92,9% der Theorie) 4(5)-Methyl-2-imidazolin vom Kp. 94° C (20 mbar). Der Umsatz ist praktisch quantitativ. Die Ausbeute bleibt auch noch nach 1500 Stunden Betrieb konstant.

Beispiel 8

Man verfährt wie bei Beispiel 1, jedoch werden 220 Teile Katalysator (Schüttgewicht 1,3 Gramm je Milliliter) Zinkoxid (Struktur analog Beispiel 1)-Aluminiumoxid (Gewichtsverhältnis 9 : 1) eingesetzt. Bei 290° C werden stündlich 75 Teile N,N'-Diformyl-1,2-diaminobutan umgesetzt. Man erhält stündlich 56,5 Teile (97% der Theorie) 4(5)-Äthyl-2-imidazolin vom Kp. 114° C (25 mbar). Der Umsatz ist praktisch quantitativ. Die Ausbeute bleibt auch noch nach 1500 Stunden Betrieb konstant.

Beispiel 9

80 Teile festes N,N'-Diformyläthylendiamin werden stündlich aus einem Vorratsgefäß zusammen mit 680 Teilen Kreisgas durch den auf 320° C erhitzten Wirbelreaktor geleitet. Der Reaktor ist mit 1500 Teilen Katalysator (Schüttgewicht 1,3 Gramm je Milliliter) Zinkoxid (Struktur analog Beispiel 1)-Aluminiumoxid (Gewichtsverhältnis 9 : 1) gefüllt. Analog Beispiel 1 erhält man stündlich 42,5 Teile (88% der Theorie) 2-Imidazolin vom Kp. 105° C (32 mbar). Das Abgas wird in einer Waschkolonne von flüchtigen Bestandteilen befreit, anschließend durch Kühlung getrocknet und als Kreisgas (Wirbel- und Verdünnungsgas) zurückgeführt. Das Kreisgas enthält 90 Gewichtsprozent CO, 8 Gewichtsprozent $CO_2$ und 2 Gewichtsprozent Kohlenwasserstoffe. Stündlich werden 17 Teile dieses Gasgemisches aus dem Kreisgas entfernt. Der Umsatz ist praktisch quantitativ. Die Ausbeute bleibt auch noch nach 1500 Stunden Betrieb konstant.

**Patentanspruch**

Verfahren zur Herstellung von 2-Imidazolinen der allgemeinen Formel I

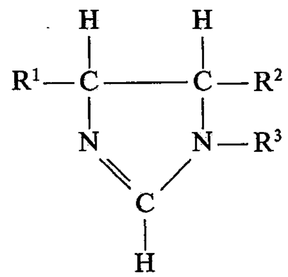

(I)

worin $R^1$, $R^2$ und $R^3$ gleich oder verschieden sein können und jeweils einen aliphatischen, araliphatischen oder aromatischen Rest oder ein Wasserstoffatom bedeuten, durch Umsetzung von N,N'-Diformylalkylendiaminen in Gegenwart von Metalloxidkatalysatoren, dadurch gekennzeichnet, daß man N,N'- Diformyl-1,2-diamine der allgemeinen Formel II

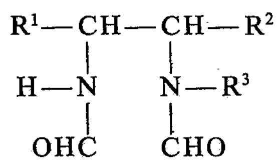

(II)

worin $R^1$, $R^2$ und $R^3$ die vorgenannte Bedeutung besitzen, bei einer Temperatur von 200 bis 350°C in Gegenwart von Inertgasen in einem Verhältnis von 5 bis 40 Mol Inertgas je Mol Ausgangsstoff II und von Zinkoxid mit einem Porenvolumen von 0,05 bis 1 Milliliter je Gramm und einer spezifischen Oberfläche von 1 bis 500 Quadratmeter je Gramm oder einem Gemisch von diesem Zinkoxid des vorgenannten Porenvolumens und der vorgenannten spezifischen Oberfläche und Aluminiumoxid oder einem Gemisch von diesem Zinkoxid des vorgenannten Porenvolumens und der vorgenannten spezifischen Oberfläche und Eisen(III)-oxid als Katalysator in der Gasphase umsetzt.

**Claim**

A process for the preparation of a 2-imidazoline of the general formula I

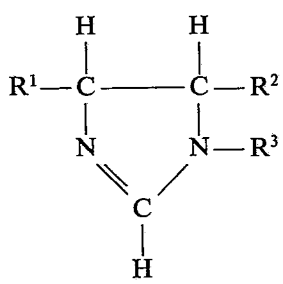

(I)

where $R^1$, $R^2$ and $R^3$ may be identical or different and each is an aliphatic, araliphatic or aromatic radical or is hydrogen, by reacting an N,N'-diformylalkylenediamine over a metal oxide catalyst, wherein an N,N'-diformyl-1,2-diamine of the general formula II

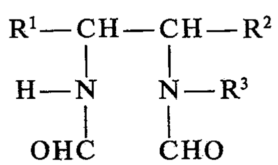

(II)

where $R^1$, $R^2$ and $R^3$ have the above meanings, is reacted in the gas phase, at from 200 to 350°C, in the presence of from 5 to 40 moles of an inert gas per mole of starting material II, over zinc oxide having a pore volume of from 0.05 to 1 milliliter per gram and a specific surface area of from 1 to 500 square meters per gram, or over a mixture of this zinc oxide having the said pore volume and specific surface

area with aluminium oxide, or over a mixture of this zinc oxide having the said pore volume and specific surface area with iron(III) oxide, as the catalyst.

## Revendication

Procédé pour la préparation de 2-imidazolines de formule générale I

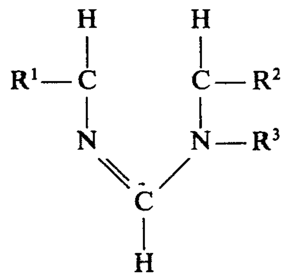

(I)

dans laquelle $R^1$, $R^2$ et $R^3$ peuvent être semblables ou différents et représentent chacun un radical aliphatique, araliphatique ou aromatique ou un atome d'hydrogène, par la mise en réaction de N,N'-diformylalcoylène-diamines en présence de catalyseurs à base d'oxydes métalliques, caractérisé en ce qu'on fait réagir en phase gazeuse des N,N'-diformyl-1,2-diamines de formule générale II

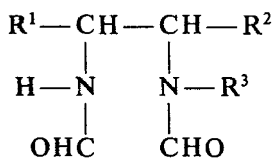

(II)

dans laquelle $R^1$, $R^2$ et $R^3$ ont les significations données ci-dessus, à une température de 200 à 350° C, en présence de gaz inertes dans un rapport de 5 à 40 mol de gaz inerte par mol de substance de départ II et d'oxyde de zinc ayant un volume de pores de 0,05 à 1 ml/g et une surface spécifique de 1 à 500 m²/g ou d'un mélange de cet oxyde de zinc, ayant le volume de pores et la surface spécifique sus-indiqués, et d'oxyde d'aluminium ou encore d'un mélange de cet oxyde de zinc, ayant le volume de pores et la surface spécifique sus-indiqués, et d'oxyde ferrique, en tant que catalyseurs.